(19) **Europäisches Patentamt European Patent Office Office européen des brevets**

(11) **EP 1 277 457 B1**

(12) **FASCICULE DE BREVET EUROPEEN**

(45) Date de publication et mention
de la délivrance du brevet:
**11.06.2008 Bulletin 2008/24**

(51) Int Cl.:
*A61Q 1/10* (2006.01)     *A61K 8/19* (2006.01)
*A61K 8/88* (2006.01)     *A61K 8/73* (2006.01)

(21) Numéro de dépôt: **02291708.2**

(22) Date de dépôt: **08.07.2002**

(54) **Mascara comprenant des particules solides**

Mascara enthaltend Feststoffpartikel

Mascara comprising solid particles

(84) Etats contractants désignés:
**AT BE BG CH CY CZ DE DK EE ES FI FR GB GR
IE IT LI LU MC NL PT SE SK TR**

(30) Priorité: **16.07.2001 FR 0109502**

(43) Date de publication de la demande:
**22.01.2003 Bulletin 2003/04**

(73) Titulaire: **L'ORÉAL**
**75008 Paris (FR)**

(72) Inventeurs:
• **Auguste, Frédéric**
**94550 Chevilly-Larue (FR)**

• **Tournilhac, Florence**
**75011 Paris (FR)**

(74) Mandataire: **Dodin, Catherine**
**L'OREAL - D.I.P.I.**
**25-29 Quai Aulagnier**
**92600 Asnières (FR)**

(56) Documents cités:
**EP-A- 1 082 953**     **WO-A-98/23251**
**FR-A- 2 794 970**

Remarques:
Le dossier contient des informations techniques présentées postérieurement au dépôt de la demande et ne figurant pas dans le présent fascicule.

**Description**

La présente invention a pour objet une composition cosmétique de revêtement des fibres kératiniques, notamment des cils ou des cheveux, comprenant des particules solides et un polymère adhérent, et son utilisation pour recourber les fibres kératiniques. La composition est destinée aux fibres kératiniques sensiblement longitudinales d'êtres humains comme les cils ou les cheveux ou bien encore aux faux-cils ou aux postiches comme les perruques. Plus spécialement, la composition est destinée au revêtement des cils.

La composition peut être une composition de maquillage, encore appelé mascara, une composition à appliquer sur un maquillage, dite encore top-coat, ou bien encore une composition de traitement des fibres kératiniques, notamment des cils. Plus spécialement, la composition est un mascara.

Le but de la présente invention est de proposer une composition de revêtement des cils conduisant après application, à un revêtement conférant un bon recourbement des cils.

Les inventeurs ont découvert qu'un tel revêtement des cils pouvait être obtenu en utilisant des particules solides particulières associées à un polymère adhérent. Il est connu des documents EP 1082953 et FR2794970 des compositions comprenant des particules de polymères filmogènes en dispersion dans une phase grasse liquide pour améliorer la tenue et/ou le non transfert des compositions ; toutefois, ces documents ne divulguent pas des compositions à teneur élevée en particules solides de dureté élevée pour améliorer le recourbement des fibres kératiniques. Le document WO98/23251 a pour objet l' amélioration de la tenue d'un mascara par incorporation de particules colloidales inorganiques.

De façon plus précise, l'invention a pour objet une composition de revêtement des fibres kératiniques, notamment des cils, comprenant, dans un milieu cosmétiquement acceptable comprenant au moins un solvant volatil, une fraction non volatile comprenant :

- un polymère apte à adhérer sur les matières kératiniques,
- des premières particules solides à 25 °C comprenant un premier matériau cristallin ou semi-cristallin solide à 25 °C présentant une transition de phase de premier ordre, de fusion ou de combustion, supérieure à 100 °C,
- et éventuellement des deuxièmes particules solides à 25 °C comprenant un deuxième matériau différent du premier matériau, les deuxièmes particules solides n'étant pas aptes à coalescer à une température inférieure ou égale à 40 °C, les premières particules solides et, le cas échéant, les deuxièmes particules solides étant présentes dans la composition en une teneur telle que la fraction volumique des premières particules solides et, le cas échéant, des deuxièmes particules solides est supérieure ou égale 60% du volume total de la fraction non volatile de la composition,

et, le cas échéant, la fraction volumique des premières particules solides étant supérieure ou égale à 30% de la fraction volumique totale des premières et deuxièmes particules solides.

L'invention a également pour objet un procédé de maquillage ou de soin non thérapeutique des fibres kératiniques, notamment des cils, comprenant l'application sur les fibres kératiniques d'une composition telle que définie précédemment.

L'invention a aussi pour objet l'utilisation d'une composition telle que définie précédemment pour recourber les fibres kératiniques, notamment les cils.

L'invention a encore pour objet l'utilisation de premières particules solides comprenant un premier matériau cristallin ou semi-cristallin solide à 25 °C présentant une transition de phase de premier ordre, de fusion ou de combustion, supérieure à 100 °C, et éventuellement de deuxièmes particules solides comprenant un deuxième matériau différent du premier matériau, les deuxièmes particules solides n'étant pas aptes à coalescer à une température inférieure ou égale à 40 °C, dans une composition de revêtement des fibres kératiniques, notamment un mascara, comprenant, dans un milieu cosmétiquement acceptable comprenant au moins un solvant volatil, une fraction non volatile comprenant un polymère apte à adhérer sur les matières kératiniques et comprenant lesdites premières et deuxièmes particules solides, les premières particules solides et, le cas échéant, les deuxièmes particules solides étant présentes dans la composition en une teneur telle que la fraction volumique des premières particules solides et, le cas échéant, des deuxièmes particules solides est supérieure ou égale 60% du volume total de la fraction non volatile de la composition, ,et, le cas échéant, la fraction volumique des premières particules solides étant supérieure ou égale à 30% de la fraction volumique totale des premières et deuxièmes particules solides, pour recourber les fibres kératiniques, notamment les cils.

On entend par particules solides des particules qui sont à l'état solide à 25 °C et à pression atmosphérique.

On entend par fraction non volatile de la composition l'ensemble des constituants présents dans la composition qui ne sont pas volatils. On entend par composé volatil un composé qui, pris isolément, a une pression de vapeur non nulle, à température ambiante (25 °C) et pression atmosphérique, allant en particulier de $10^{-2}$ à 300 mm de Hg (1,33 Pa à 40.000 Pa) et de préférence supérieure à 0,3 mm de Hg (40 Pa).

La fraction non volatile de la composition correspond en fait au mélange des constituants restant sur les cils après le séchage complet du mascara appliqué sur les cils.

Pour obtenir un bon recourbement des cils, la composition selon l'invention comprend des particules solides, dites premières particules, comprenant (en particulier formé de) un matériau, appelé premier matériau, cristallin ou semi-cristallin solide à température ambiante (25 °C) présentant une transition de phase de premier ordre, de fusion (passage de l'état solide à l'état liquide) ou de combustion, supérieure à 100 °C, de préférence supérieure à 120 °C, et mieux supérieure à 150 °C. La température de fusion ou de combustion du premier matériau peut être mesurée selon la norme ASTM E794-98.

Par "matériau semi-cristallin", on entend au sens de l'invention, un matériau, notamment un polymère, comportant une partie cristallisable et une partie amorphe présentant une température de changement de phase réversible du premier ordre, en particulier de fusion (transition solide-liquide).

Avantageusement, le premier matériau desdites particules présente une dureté Vickers supérieure ou égale à 10, notamment allant de 10 à 7500, de préférence supérieure ou égale à 200, notamment allant de 200 à 7500, et mieux supérieure ou égale à 400, notamment alant de 400 à 7500.

La dureté VICKERS (HV) est déterminée en appliquant sur le matériau un pénétromètre en forme de pyramide à base carrée, à l'aide d'une charge P. On mesure ensuite la dimension moyenne d'une diagonale de l'empreinte carrée obtenue avec le pénétromètre.
La dureté VICKERS (HV) est alors calculée par la relation :

$$HV = \frac{1854,4 \times P}{d^2}$$

d = diagonale moyenne en $\mu$m

P = charge appliquée en g

La mesure de la dureté VICKERS peut être effectuée à l'aide du microduromètre M 400 g 2 de la société LECO.

Le premier matériau desdites premières particules peut être un matériau minéral qui peut être choisi parmi la silice, le verre, le diamant, le cuivre, le nitrure de bore, les céramiques, les oxydes métalliques, notamment les oxydes de fer comme l'oxyde de fer noir, l'oxyde de fer rouge, l'oxyde de fer jaune, les oxydes de titane, les micas, l'alumine, ou bien encore un polymère comme les polyamides par exemple le nylon, et leurs mélanges.

Les dites premières particules peuvent être des particules pleines, ou bien encore des particules creuses. Par exemple, on peut utiliser la silice creuse vendue sous la dénomination « SUNSIL-130 » par la société SUNJIN CHEMICAL.

Selon un premier mode de réalisation de la composition selon l'invention, lesdites premières particules sont formées essentiellement dudit premier matériau défini précédemment.

Selon un deuxième mode de réalisation de la composition selon l'invention, ledistes premières particules solides comprennent, voire sont formées essentiellement de, au moins deux premiers matériaux différents. C'est par exemple le cas des micas recouverts d'oxyde de titane ou d'oxyde de fer.

Selon un troisième mode de réalisation de la composition selon l'invention, lesdites premières particules solides comprennent au moins ledit premier matériau, et au moins un matériau additionnel, différent dudit premier matériau, ledit premier matériau formant la surface desdites premières particules. Pour ces particules solides, ledit premier matériau ayant les caractéristiques décrites précédemment se trouve à la surface desdites premières particules, ces dernières comprenant un matériau additionnel recouvert par le premier matériau.

Avantageusement, les dites premières particules solides peuvent avoir une taille moyenne allant de 5 nm à 50 $\mu$m, et de préférence allant de 20 nm à 50 $\mu$m.

La composition selon l'invention peut comprendre, en plus des premières particules solides décrites précédemment, d'autres particules solides, appelées deuxièmes particules solides, différentes des premières particules solides.
Ces deuxièmes particules correspondent aux particules solides à 25 °C de tout matériau, différent des premières particules, restant sous forme de particules individualisées, ou éventuellement collées mais qui conservent dans ce cas leur état de particule individuelle (ces particules collées ne sont pas coalescées à une température inférieure ou égale à 40 °C).

En particulier, les deuxièmes particules solides peuvent être choisies parmi :

- des particules solides à 25 °C, appelées deuxièmes particules solides primaires, comprenant (en particulier formé de) un matériau amorphe ayant une température de transition vitreuse supérieure ou égale à 60 °C,
- d'autres particules solides à 25 °C, appelées deuxièmes particules solides secondaires, différentes desdites deuxièmes particules primaires,
- et leurs mélanges.

Les deuxièmes particules solides primaires comprennent (en particulier sont formées de) un matériau amorphe, en particulier un polymère, ayant une température de transition vitreuse supérieure ou égale à 60 °C (notamment allant de 60 °C à 800 °C), avantageusement supérieure ou égale à 80 °C (notamment allant de 80 °C à 700 °C), et de préférence supérieure ou égale à 100 °C (notamment allant de 100 °C à 500 °C). La température de transition vitreuse peut être mesurée par DSC (Differential Scanning Calorimetry) selon la norme ASTM D3418-97.

Comme matériau amorphe, on peut utiliser un polymère non filmogène à une température inférieure ou égale à 40 °C ayant une température de transition vitreuse telle que décrite précédemment.
On entend par « polymère non filmogène à une température inférieure ou égale à 40 °C» un polymère qui n'est pas apte à former à lui seul, ou en présence d'agent auxiliaire de filmification, un film continu et adhérent sur un support, notamment sur les matières kératiniques, à une température inférieure ou égale à 40 °C.

On entend par agent auxiliaire de filmification les agents plastifiants et les agents de coalescence connus de l'homme du métier pour favoriser la filmification des polymères.

Comme polymère amorphe ayant une température de transition vitreuse supérieure ou égale à 60 °C, on peut utiliser des polymères radicalaires ou les polycondensats ayant la température de transition vitreuse définie.

Comme polymère radicalaire, on peut citer :

- les polymères d'éthylène, notamment de cycloéthylène, de naphtyléthylène ;
- les polymères de propylène, notamment d'hexafluoropropylène ;
- les polymères acryliques, notamment les polymères d'acide acrylique, d'acrylate de diméthyl-adamanthyl, de chloroacrylate ;
- les polymères d'acrylamide ;
- les polymères de (méth)acrylonitrile ;
- les polymères d'acétylstyrène, de carboxystyrène, de chlorométhylstyrène ;

Comme polycondensats, on peut citer les polycarbonates, les polyuréthanes, les polyesters, les polyamides, les polysulfones, les polysulfonamides, les carbohydrates comme l'amylose triacétate.

Les deuxièmes particules solides primaires peuvent être des particules pleines ou des particules creuses.

Selon un premier mode de réalisation, les deuxièmes particules solides primaires sont formées essentiellement dudit matériau amorphe décrit précédemment.

Selon un deuxième mode de réalisation, les deuxièmes particules solides primaires comprennent au moins ledit matériau amorphe et au moins un matériau additionnel, différent du matériau amorphe, ledit matériau amorphe formant la surface,

ou l'écorce, desdites deuxièmes particules solides primaires et le matériau additionnel formant l'intérieur, ou le coeur, desdites deuxièmes particules solides primaires.

Le matériau additionnel peut être par exemple un polymère additionnel ayant une température de transition vitreuse inférieure à 60 °C, et de préférence inférieure à 45 °C.

Ainsi, les deuxièmes particules solides primaires peuvent être par exemple des particules coreshell de polymères, comprenant une partie externe (c'est-à-dire une écorce) formée du premier matériau amorphe ayant une température de transition vitreuse supérieure ou égale à 60 °C et comprenant une partie interne (c'est-à-dire un coeur) ayant une température de transition vitreuse inférieure à 60 °C.

Avantageusement, la teneur du matériau amorphe dans les deuxièmes particules solides primaires est telle que la fraction volumique du matériau amorphe est supérieure ou égale à 10 %, et de préférence supérieure ou égale à 30 %, en volume du volume total des deuxièmes particules solides primaires.

Les deuxièmes particules solides primaires peuvent avoir une taille moyenne allant de 10 nm à 50 $\mu$m, et de préférence allant de 20 nm à 1 $\mu$m.

Comme deuxièmes particules solides primaires, on peut utiliser les dispersions aqueuses de polymère non filmogène vendues sous les dénominations « JONCRYL® SCX 8082 » , «JONCRYL® 90 » par la société JOHNSON POLYMER, « NEOCRYL® XK 52» par la société AVECIA RESINS, « RHODOPAS® 5051 » par la société RHODIA CHIMIE.

Toutes les constituants présents dans la composition selon l'invention se trouvant à l'état de particules solides à 25 °C et qui ne coalescent pas à une température inférieure ou égale à 40 °C, à eux seuls ou en présence des autres constituants présents dans la composition, sont considérées comme étant soit des premières particules solides ou soit des deuxièmes particules solides selon les définitions décrites précédemment.

Ainsi, par exemple, les deuxièmes particules secondaires peuvent être en un matériau choisi parmi les cires, les charges, les polymères différents du matériau amorphe présent dans les deuxièmes particules solides primaires décrites précédemment.

Les additifs décrits ci-après, lorsqu'ils sont sous la forme de particules solides à 25 °C, sont considérés comme étant soit des premières particules solides, soit des deuxièmes particules solides telles que décrites précédemment lorsque ces additifs possèdent les caractéristiques correspondantes définies précédemment.

En particulier, le polymère adhérent présent dans la composition selon l'invention peut être sous la forme de particules solides. Dans ce cas, ces particules sont considérées comme étant des particules solides telles que définies précédemment si ce polymère possède les caractéristiques définies précédemment.

Dans la composition selon l'invention, les dites premières et, le cas échéant les deuxièmes particules solides sont présentes en une teneur telle que la fraction volumique des premières particules solides et, le cas échéant, des deuxièmes particules solides est supérieure ou égale à 50 % du volume total de la fraction non volatile de la composition, ce qui signifie que le volume total de toutes les premières particules et, le cas échéant, des deuxièmes particules représente au moins 50 % (notamment va de 50 % à 99 %) du volume total de la fraction non volatile de la composition.

On entend par « fraction volumique des premières particules solides et, le cas échéant, des deuxièmes particules solides» le pourcentage du volume total de toutes les premières particules solides et, le cas échéant, de toutes les deuxièmes particules solides présentent dans la fraction non volatile de la composition, par rapport au volume total de tous les composés de la fraction non volatile de la composition.

Ladite fraction volumique des premières particules solides et, le cas échéant, des deuxièmes particules solides, est supérieure ou égale à 60 % (notamment va de 60 % à 99 %), et de préférence supérieure ou égale à 70 % (notamment va de 70 % à 95 %) du volume total de la fraction non volatile de la composition.

La fraction volumique (FV) de particules solides présente dans la fraction non volatile de la composition est égale au pourcentage du volume total V desdites particules divisé par le volume total V' de la fraction non volatile de la composition.

Le volume V de particules solides est égal à la masse m desdites particules solides dans la composition divisé par la masse volumique Mv des particules. La masse volumique est calculée selon la méthode décrite ci-après.

$$\text{Fraction volumique : } FV = 100 \times V / V'$$

et $V = m/Mv$

Le volume total V' de la fraction non volatile de la composition est calculé en additionnant le volume de chaque constituant non volatil présent dans la composition.

Avantageusement, lorsque la composition comprend des deuxièmes particules telles que définies précédemment, les premières particules sont présentes dans la composition en une teneur telle que la fraction volumique des premières particules solides est supérieure ou égale à 30% de la fraction volumique totale des premières et deuxièmes particules solides, notamment allant de 30% à 100 %, avantageuseument supérieure ou égale à 40 notamment allant de 40 % à 100 %, et mieux supérieure ou égale à 50 %, notamment allant de 50 % à 100 %.

En particulier, lorsque la composition comprend les deuxièmes particules solides primaires telles que définies précédemment, la fraction volumique desdites premières particules solides et desdites deuxièmes particules solides primaires, est supérieure ou égale à 10,05 % (notamment allant de 10,05 % à 100 %) du volume total des premières et deuxièmes particules solides, avantageusement supérieure ou égale à 20,05 % (notamment allant de 20,05 % à 100 %), et de préférence supérieure ou égale à 30,05 % (notamment allant de 30,05 % à 100 %), préférentiellement supérieure ou égale à 40,05 % (notamment allant de 40,05 % à 100 %), et mieux supérieure ou égale à 50 % (notamment allant de 50,05 % à 100 %).

Le solvant volatil présent dans la composition selon l'invention peut être choisi parmi l'eau, les solvants organiques volatils et les huiles volatiles définis ci-après, et leurs mélanges.

Dans la présente demande, on entend par « polymère apte à adhérer sur les matières kératiniques », appelé par la suite polymère adhérent, un polymère apte à rester fixé sur les matières kératiniques telles que les fibres kératiniques comme les cils , les-cheveux, ou la peau, et de préférence sur les cils, lors du contact du polymère avec lesdites matières kératiniques. Un tel polymère adhérent a d'ailleurs une bonne aptitude à former un dépôt sur les matières kératiniques et reste fixé sur ces dernières.

Avantageusement, le polymère adhérent peut être un polymère filmogène à une température inférieure ou égale à 40 °C. Dans la présente demande, on entend par "polymère filmogène", un polymère apte à former à lui seul ou en présence d'un agent auxiliaire de filmification, un dépôt, notamment un film, continu et adhérent sur un support, notamment sur les matières kératiniques.

Le polymère adhérent présent dans la composition selon l'invention peut être un polymère solubilisé ou dispersé sous forme de particules solides dans une phase aqueuse de la composition ou bien encore solubilisé ou dispersé sous forme de particules solides dans une phase grasse liquide. La composition peut comprendre un mélange de ces polymères. Lorsque le polymère adhérent se présente sous forme de particules solides, ces particules peuvent présenter une taille moyenne de particules allant de 5 nm à 10 $\mu$m, notamment allant de 5 nm à 5 $\mu$m, avantageusement allant de 5 nm à 600 nm, et de préférence allant de 20 nm à 300 nm.

Le polymère adhérent peut être présent dans la composition selon l'invention en une teneur en matières sèches allant de 0,1 % à 50 % en poids par rapport au poids total de la composition, de préférence de 0,5 % à 40 % en poids, et mieux de 1 % à 30 % en poids.

Parmi les polymères adhérents utilisables dans la composition de la présente invention, on peut citer les polymères synthétiques, de type radicalaire ou de type polycondensat, les polymères d'origine naturelle, et leurs mélanges.

Par polymère radicalaire, on entend un polymère obtenu par polymérisation de monomères à insaturation notamment éthylénique, chaque monomère étant susceptible de s'homopolymériser (à l'inverse des polycondensats). Les polymères de type radicalaire peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$. Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Il est possible d'utiliser tout monomère connu de l'homme du métier entrant dans les catégories de monomères acryliques et vinyliques (y compris les monomères modifiés par une chaîne siliconée).

Parmi les polycondensats, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyétherpolyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO$_3$M, avec M représentant un atome d'hydrogène, un ion ammonium NH$_4^+$ ou un ion alcalin, alcalino-terreux ou métallique, comme par exemple un ion Na$^+$, Li$^+$, K+, Mg$^{2+}$, Ca$^{2+}$, Cu$^{2+}$, Fe$^{2+}$, Fe$^{3+}$. On peut utiliser notamment un monomère aromatique bifonctionnel comportant un tel groupement -SO$_3$M.

Le noyau aromatique du monomère aromatique bifonctionnel portant en outre un groupement -SO$_3$M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO$_3$M : l'acide sulfoisophtalique, l'acide sulfotéréphtalique, l'acide sulfophtalique, l'acide 4-sulfonaphtalène-2,7-dicarboxylique. On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ® par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère adhérent peut être présent sous la forme de particules solides en dispersion aqueuse, connue généralement sous le nom de latex ou pseudolatex. Les techniques de préparation de ces dispersions sont bien connues de l'homme du métier.

Comme dispersion aqueuse de polymère adhérent, on peut utiliser les dispersions acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, DAITOSOL 5000 AD® par la société DAITO KASEY KOGYO; ou bien encore les dispersions aqueuses de polyuréthane vendues sous les dénominations NEOREZ R-981®, NEOREZ R-974® par la société AVECIA-NEORESINS, les AVALURE UR-405®, AVALURE UR-410®, AVALURE UR-425 ®, AVALURE UR-450®, SANCURE 875®, SANCURE 861®, SANCURE 878®, SANCURE 2060® par la société GOODRICH, IMPRANIL 85® par la société BAYER, AQUAMERE H-1511® par la société HYDROMER.

Comme dispersion aqueuse de polymère adhérent, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Selon une deuxième variante de réalisation de la composition selon l'invention, le polymère adhérent peut être un polymère hydrosoluble et est donc présent dans la phase aqueuse de la composition sous forme solubilisée. Comme exemples de polymères filmogènes hydrosolubles, on peut citer

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :

  • les gommes arabiques, la gomme de guar, les dérivés du xanthane, la gomme de karaya ;
  • les alginates et les carraghénanes ;
  • les glycoaminoglycanes, l'acide hyaluronique et ses dérivés ;
  • la résine shellac, la gomme de sandaraque, les dammars, les élémis, les copals ;
  • l'acide désoxyribonucléïque ;
  • les muccopolysaccharides tels que l'acide hyaluronique, les chondroïtines sulfate, et leurs mélanges.

Selon une autre variante de réalisation de la composition selon l'invention, le polymère adhérent peut être présent dans une phase grasse liquide comprenant des huiles ou solvants organiques tels que ceux décrits précédemment. Par "phase grasse liquide", on entend, au sens de l'invention, une phase grasse liquide à température ambiante (25°C) et pression atmosphérique (760 mm de Hg, soit 105 Pa), composée d'un ou plusieurs corps gras liquides à température ambiante, appelés aussi huiles, généralement compatibles entre eux.

De préférence, la phase grasse liquide comprend une huile volatile, éventuellement en mélange avec une huile non volatile, les huiles pouvant être choisies parmi les huiles citées ci-après.

Selon un troisième mode de réalisation de la composition selon l'invention, le polymère adhérent peut être présent sous forme de particules, stabilisées en surface, dispersées dans la phase grasse liquide.

La dispersion de particules de polymère stabilisées en surface peut être fabriquée comme décrit dans le document EP-A-749747.

Les particules de polymère sont stabilisées en surface grâce à un stabilisant qui peut être un polymère séquencé, un polymère greffé, et/ou un polymère statistique, seul ou en mélange.

Des dispersions de polymère filmogène dans la phase grasse liquide, en présence d'agent stabilisants, sont notamment décrites dans les documents EP-A-749746, EP-A-923928, EP-A-930060 dont le contenu est incorporé à titre de référence dans la présente demande.

La taille des particules de polymères en dispersion soit dans la phase aqueuse, soit dans la phase grasse liquide, peut aller de 5 nm à 10 $\mu$m, notamment aller de 5 nm à 5 $\mu$m, avantageusement aller de 5 nm à 600 nm, et de préférence aller de 20 nm à 300 nm.

Selon un quatrième mode de réalisation de la composition selon l'invention, le polymère adhérent peut être solubilisé dans la phase grasse liquide, on dit alors que le polymère filmogène est un polymère liposoluble.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyl, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % dé divinyl benzène et propionate d'allyle/stéarate d'allyle réticulé avec 0,2 % de divinyl benzène.

Comme polymères liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

La composition selon l'invention peut comprendre un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

Selon un mode de réalisation préféré de la composition selon l'invention, le polymère adhérent peut être un polymère apte à former un dépôt, notamment un film, produisant à une concentration de 7 % dans l'eau, une rétraction du stratum corneum isolé de plus de 1 % à 30°C sous une humidité relative de 40 %, de préférence de plus de 1,2 %, et mieux de plus de 1,5 %. Cette rétraction est mesurée à l'aide d'un extensiomètre, selon la méthode décrite ci-après.

Le solvant volatil présent dans la composition peut être choisi parmi l'eau ou les composés organiques volatils, ou leur mélange.

Selon un premier mode de réalisation de la composition selon l'invention, la composition peut comprendre un milieu aqueux, constituant une phase aqueuse, qui peut être la phase continue de la composition.

La phase aqueuse peut comprendre, voire être constituée essentiellement, de l'eau ; elle peut également comprendre un mélange d'eau et de solvant organique miscible à l'eau (solvant apte à former avec l'eau un mélange homogène et transparent à l'oeil à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$.

La phase aqueuse (eau et éventuellement le solvant organique miscible à l'eau) peut être présente, en une teneur allant de 0,1 % à 98 % en poids, par rapport au poids total de la composition, de préférence de 1 % à 80 % en poids, et mieux de 5 % à 65 % en poids.

Selon un deuxième mode de réalisation de la composition selon l'invention, la composition peut comprendre au moins une huile ou solvant organique volatile qui peut notamment former une phase grasse, et en particulier une phase grasse continue. La composition peut être une composition anhydre.

Par " huile volatile ou solvant organique volatile", on entend au sens de l'invention des solvants organiques et des huiles cosmétiques volatiles, liquides à température ambiante, ayant une pression de vapeur non nulle, à température ambiante et pression atmosphérique, allant en particulier de $10^{-2}$ à 300 mm de Hg (1,33 Pa à 40.000 Pa) et de préférence supérieure à 0,3 mm de Hg (40 Pa). Par "huile non volatile", on entend une huile ayant une pression de vapeur inférieure à $10^{-2}$ mm de Hg (1,33 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en $C_8$-$C_{16}$ le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité $\leq$ 8 centistokes (8 $10^{-6}$ m$^2$/s), et ayant notamment de 2 à 7 atomes de silicium, ces silicones comportant éventuellement des groupes alkyle

ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

Comme solvant fluoré volatil, on peut utiliser le perfluorométhyl-cyclopentane ou le 1,1,1,2,2,3,4,5,5,5-décafluoropentane.

L'huile volatile peut être présente dans la composition selon l'invention en une teneur allant de 0,1 % à 98 % en poids, par rapport au poids total de la composition, de préférence allant de 1 % à 65 % en poids, et mieux allant de 5 % à 65 % en poids.

La composition peut également comprendre au moins une huile non volatile, et notamment choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,

- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_5 + R_6$ soit ≥ 10, comme par exemple l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- le dicaprylyl carbonate vendu sous la dénomination « CETIOL CC » par la société COGNIS ;

et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Les huiles non volatiles peuvent être présentes dans la composition selon l'invention en une teneur allant de 0,1 % à 50 % en poids, de préférence de 0,1 % à 30 % en poids, par rapport au poids total de la composition, et mieux de 0,1 % à 20 % en poids.

La composition selon l'invention peut comprendre en outre au moins une cire.

Par "cire", on entend au sens de la présente invention, un composé gras lipophile, solide à température ambiante (25 °C) et pression atmosphérique (760 mm de Hg, soit 105 Pa), à changement d'état solide/liquide réversible, ayant une température de fusion allant de 30 °C à 99 °C, et mieux allant de 45 °C à 99 °C.

En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER , avec une montée en température de 5 ou 10 °C par minute.

Les cires, au sens de l'invention, sont celles généralement utilisées dans les domaines cosmétique et dermatologique . On peut notamment citer la cire d'abeilles, la cire de lanoline, et les cires d'insectes de Chine; la cire de riz, la cire de Carnauba, la cire de Candellila, la cire d'Ouricury, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40°C et mieux à plus de 55°C,.

On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée. On peut encore citer les cires de silicone ou les cires fluorées.

Les cires présentes dans la composition peuvent être dispersées sous forme de particules, notamment dans un milieu aqueux. Ces particules peuvent avoir une taille moyenne allant de 50 nm à 50 μm, et de préférence de 50 nm à 10 μm.

En particulier, la cire peut être présente sous forme d'émulsion cires-dans-eau, les cires pouvant être sous forme de particules de taille moyenne allant de 1 μm à 50 μm, et de préférence de 1 μm à 10 μm.

Dans un autre mode de réalisation de la composition selon l'invention, la cire peut être présente sous forme de microdispersion de cire, la cire étant sous forme de particules dont la taille moyenne est inférieure à 1 μm, et va notamment de 50 nm à 500 nm. Des microdispersions de cires sont décrites dans les documents EP-A-557196, EP-A-1048282.

La cire peut être présente dans la composition selon l'invention en une teneur allant de 0,1 % à 50 % en poids, par rapport au poids total de la composition, de préférence de 0,5 % à 30 % en poids, et mieux de 1 % à 20 % en poids.

La composition selon l'invention peut contenir des agents tensioactifs émulsion-nants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :

- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de $C_1$-$C_6$ alkyl glucose polyoxyéthylénés, et leurs mélanges.
- parmi les tensioactifs anioniques : les acides gras en $C_{16}$-$C_{30}$ neutralisés par les amines, l'ammoniaque ou les sels alcalins, et leurs mélanges ; le copolymère acide acrylique /itaconatee de monostéaryleoxyéthyléné (20 OE) en dispersion aqueuse à 30 % en poids vendu sous la dénomination « STRUCTURE 2001 » par la société National Starch, le copolymère acide acrylique/itaconate de monocétyle éthoxylé (20 OE) en dispersion aqueuse à 30 % vendu sous la dénomination « STRUCTURE 3001 » par la société National Starch.

On utilise de préférence des tensioactifs permettant l'obtention d'émulsion huile-dans-eau ou cire-dans-eau.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 50 % en poids, par rapport au poids total de la composition, de préférence allant de 0,01 % à 30 % en poids.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

La composition de l'invention peut comprendre, en outre, tout additif usuellement utilisé en cosmétique tels que les antioxydants, les charges, les conservateurs, les parfums, les neutralisants, les épaississants, les agents de coalescence, les plastifiants, les actifs cosmétiques ou dermatologiques comme par exemple des émollients, des hydratants, des vitamines, des filtres solaires, et leurs mélanges. Ces additifs peuvent être présents dans la composition en une teneur allant de 0,01 à 20 % du poids total de la composition et mieux de 0,01 à 10% (si présents).

La composition selon l'invention peut se présenter sous la forme d'émulsion huile-dans-eau, eau-dans-huile, de dispersion cire-dans-eau ou bien encore être une composition anhydre.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique ou dermatologique.

**Méthode de mesure de la masse volumique de particules solides :**

La masse volumique apparente de particules solides est mesurée à l'aide d'un pycnomètre Gay-Lussac.

On utilise une balance de précision (précision de 1 mg) et les mesures sont effectuées dans une enceinte thermostatée à 25 °C ( ± 0.5°C). On utilise également deux liquides de référence ayant une masse volumique Mv qui sont l'eau déminéralisée (MV = 1000 kg/m$^3$) et l'heptane (MV = 683.7 kg/m$^3$). On effectue la mesure de la masse volumique des particules solides avec chaque liquide de référence.

On place le pycnomètre et les produits utilisé pour effectuer la mesure à la température de 25°C. Les masses citées ci-après sont exprimées en kilogrammes.

On mesure la masse M0 du pycnomètre, puis on remplit complètement le pycnomètre du liquide de référence employé, en évitant l'introduction de bulle d'air. On mesure la masse M1 du pycnomètre rempli.

Puis on prépare un mélange de masse M2 du matériau dont on veut mesurer sa masse volumique Mv2 avec une masse M3 de liquide de référence. On agite le mélange puis juste à la fin de l'agitation, on remplit le pycnomètre avec ce mélange et on mesure la masse M4 du pycnomètre rempli. On détermine ainsi la masse M4 - M0 du mélange présent dans le pycnomètre.

Le pycnomètre ayant un volume de remplissage constant, on peut donc établir la relation suivante : (M1-M0) / Mv = (M2/Mv2 + M3/Mv) x (M4-M0) / (M2+M3)

Cette relation permet de calculer la valeur de la masse volumique Mv2 des particules solides, exprimée en kg/m$^3$. On détermine ainsi pour chacun des liquides de référence une valeur de la masse volumique des particules solides. Selon l'invention, la valeur la plus élevée (parmi la masse volumique mesurée avec l'eau distillée et la masse volumique mesurée avec l'heptane) est retenue comme valeur de la masse volumique pour la détermination de la fraction volumique des particules solides.

**Méthode de mesure de rétraction d'un polymère :**

Le principe consiste à mesurer avant traitement et après traitement la longueur d'une éprouvette de stratum cornéum isolé et de déterminer le pourcentage de rétraction de l'éprouvette.

On utilise des éprouvettes de 1 cm X 0,4 cm de stratum cornéum d'épaisseur allant de 10 à 20 $\mu$m disposées sur l'extensiomètre MTT 610 commercialisé par la société DIASTRON.

L'éprouvette est placée entre 2 mâchoires puis laissée pendant 12 heures dans une atmosphère à 30 °C et 40 % d'humidité relative.

On tracte à la vitesse de 2 mm/minute l'éprouvette d'une longueur comprise entre 5 et 10 % de la longueur initiale pour déterminer la longueur $l_1$ à partir de laquelle l'éprouvette commence à exercer une force sur les mâchoires et détectée par l'appareil.

On détend ensuite l'éprouvette puis on applique sur le stratum cornéum 2 mg d'une composition aqueuse à 7 % en poids de polymère. Après évaporation totale de l'eau de la composition, on tracte l'éprouvette dans les mêmes conditions que celles décrites précédemment pour déterminer également la longueur $l_2$ pour l'éprouvette traitée.

Le pourcentage de rétraction est déterminé par le rapport : 100 X $(l_2 - l_1)/l_1$.

L'invention est illustrée plus en détail dans les exemples suivants.

**Exemples 1 et 2 comparatifs :**

On a préparé une composition de mascara (exemple 1) comprenant :

- oxyde fer noir (Sicovit noir 85E172 de BASF)        35 g
- propylène glycol        5 g
- hydroxyéthylcellulose        1 g (Cellosize QP4400M d'Amerchol)
- eau qsp        100 g

Ce mascara ne fait pas partie de l'invention telle que revendiquée

La fraction non volatile de ce mascara contient une fraction volumique de particules solides (oxyde de fer noir) de 54 % (par rapport au volume total de la fraction non volatile) et 100 % des particules solides sont des premières particules telles que définies dans la présente invention.

On a également préparé une composition de mascara ne faisant pas partie de l'invention (exemple 2) comprenant :

- oxyde fer noir (Sicovit noir 85E172 de BASF)        5 g
- propylène glycol        5 g
- hydroxyéthylcellulose (Cellosize QP4400M d'Amerchol)        1 g
- eau qsp        100 g

La fraction non volatile de ce mascara contient une fraction volumique de particules solides (oxyde de fer noir) de 14,3 % (par rapport au volume total de la fraction non volatile) et 100 % des particules solides sont des premières particules telles que définies dans la présente invention.

On a mesuré les propriétés recourbantes de ces 2 mascaras selon le protocole suivant :

On a utilisé des éprouvettes de cheveux caucasiens comprenant 15 cheveux d'une longueur de 15 mm présentant un arc de courbure ayant un rayon de courbure compris entre environ 6 et 7 mm. Ces éprouvettes sont fixées sur un support de telle façon que le dessus de l'éprouvette correspond au côté interne de l'arc formé par l'éprouvette, le dessous de l'éprouvette correspondant au côté externe de l'arc formé par l'éprouvette. Avant l'application du mascara, la courbure de l'éprouvette de cheveux a été mesurée en prenant une photo numérique de profil à l'aide du Macrozoom Navitar.

Puis on appliqué le mascara sur chaque éprouvette à l'aide d'une brosse sur le dessous de l'éprouvette. On a effectué 3 séries de 10 passages de la brosse avec une attente de 2 minutes entre chaque série de 10 passages. 20 minutes après le dernier passage de la brosse sur l'éprouvette, on a photographié l'éprouvette de cheveux maquillés.

Les images sont traitées avec le système d'analyse d'image Microvision et on détermine le rayon de courbure moyens des cheveux avant maquillage (Rc i) et le rayon de courbure moyen des cheveux après maquillage (Rcf), le rayon de courbure étant mesuré en millimètre.

Le recourbement R est calculé selon la formule :

$$R = 1/R_{ci} - 1/R_{cf}$$

Plus la valeur de R est grande, plus le recourbement des cils mesuré est important.

On a obtenu les résultats suivants :

Exemple 1 (invention) : R = 0,010 mm$^{-1}$

Exemple 2 (hors invention) : R = 0,002 mm$^{-1}$

On a ainsi constaté que les propriétés recourbantes du mascara de l'exemple 1 sont supérieures à celles du mascara de l'exemple 2. Une plus grande quantité de particules solides d'oxyde de fer noir présente dans le mascara permet d'augmenter le recourbement des cils.

**Exemples 3 et 4 comparatifs :**

On a préparé une composition de mascara selon l'invention (exemple 3) comprenant :

- poudre de nylon-12 (Orgasol 2002 d'Atochem)          30 g
- oxyde fer noir (Sicovit noir 85E172 de BASF)          5 g
- propylène glycol          5 g
- hydroxyéthylcellulose          1 g (Cellosize QP4400M d'Amerchol)
- eau qsp          100 g

La fraction non volatile de ce mascara contient une fraction volumique de particules solides (nylon, oxyde de fer noir) de 90 % (par rapport au volume total de la fraction non volatile) et 100 % des particules solides (nylon, oxyde de fer noir) sont des premières particules telles que définies dans la présente invention.

On a également préparé une composition de mascara ne faisant pas partie de l'invention (exemple 4) comprenant :

- Cire de carnauba          30 g
- Oxyde de fer noir (Sicovit noir 85E172 de BASF)          5 g
- propylène glycol          5 g
- hydroxyéthylcellulose          1 g (Cellosize QP4400M d'Amerchol)
- Tensioactif (Brij 35)          3 g
- eau qsp          100 g

Pour fabriquer ce mascara, on a d'abord préparé une dispersion aqueuse de cire de carnauba en mélangeant à 95 °C, 40 g de cire de carnauba, 4 g de tensioactif alcool laurylique polyoxyéthyléné à 23 motifs d'oxyde d'éthylène vendu sous la dénomination « BRIJ 35 » par la société UNICHEMA et 56 g d'eau chauffée à 95 °C, sous agitation à l'aide d'un agitateur Ultraturrax, jusqu'à obtenir une dispersion aqueuse de cire ayant une taille moyenne de particules d'environ 300 nm.

Puis on a mélangé 75 g de la dispersion de cire avec la fraction aqueuse complémentaire comprenant les autres ingrédients.

La fraction non volatile de ce mascara contient une fraction volumique de particules solides (cire de carnauba, oxyde de fer noir) de 84 % (par rapport au volume total de la fraction non volatile) et 3,3 % des particules solides (oxyde de fer noir) sont des premières particules telles que définies dans la présente invention.

On a mesuré les propriétés recourbantes de ces 2 mascaras selon le protocole décrit aux exemples 1 et 2 et on a obtenu les résultats suivants :

Exemple 3 (invention) : R = 0,016 mm$^{-1}$

Exemple 4 (hors invention) : R = 0,012 mm$^{-1}$

Les propriétés recourbante du mascara de l'exemple 3 selon l'invention sont supérieures à celles du mascara de l'exemple 4 ne faisant pas partie de l'invention. La présence de particules de nylon à la place des particules de cire de carnauba permet d'améliorer le recourbement des cils.

## Revendications

1. Composition de revêtement des fibres kératiniques comprenant, dans un milieu cosmétiquement acceptable comprenant au moins un solvant volatil ayant une pression de vapeur non nulle, à température ambiante et pression atmosphérique, une fraction non volatile comprenant :

   - un polymère apte à adhérer sur les matières kératiniques,
   - des premières particules solides comprenant un premier matériau cristallin ou semi-cristallin solide à 25 °C présentant une température de transition de phase de premier ordre, de fusion ou de combustion, supérieure à 100 °C,
   - et éventuellement des deuxièmes particules solides comprenant un deuxième matériau différent du premier matériau, les deuxièmes particules solides n'étant pas aptes à coalescer à une température inférieure ou égale à 40 °C, les premières particules solides et, le cas échéant, les deuxièmes particules solides étant présentes dans la composition en une teneur telle que la fraction volumique des premières particules solides et, le cas échéant, des deuxièmes particules solides est supérieure ou égale à 60 % du volume total de la fraction non volatile de la composition,

   et, le cas échéant, la fraction volumique des premières particules solides étant supérieure ou égale à 30 % de la fraction volumique totale des premières et deuxièmes particules solides.

2. Composition selon la revendication 1, **caractérisée par le fait que** le premier matériau cristallin ou semi-cristallin présente une transition de phase de premier dre supérieure à 120 °C, de préférence supérieure à 150 °C.

3. Composition selon l'une des revendications 1 ou 2, **caractérisée par le fait que** le premier matériau a une dureté Vicker supérieure ou égale à 10, de préférence allant de 10 à 7500.

4. Composition selon l'une quelconque des revendications 1 à 3, **caractérisée par le fait que** le premier matériau a une dureté Vicker supérieure ou égale à 200, de préférence allant de 200 à 7500.

5. Composition selon l'une quelconque des revendications 1 à 4, **caractérisée par le fait que** le premier matériau a une dureté Vicker supérieure ou égale à 400, de préférence allant de 400 à 7500.

6. Composition selon l'une quelconque des revendications précédentes, **caractérisée par le fait que** le premier matériau est choisi dans le groupe formé par la silice, le verre, le diamant, le cuivre, le nitrure de bore, les céramiques, les oxydes métalliques, les micas, les polyamides, et leurs mélanges.

7. Composition selon la revendication 6, **caractérisée par le fait que** premier matériau est choisi parmi les oxydes de fer.

8. Composition selon l'une quelconque des revendications précédentes, **caractérisée par le fait que** les premières particules solides ont une taille moyenne allant de 5 nm à 50 $\mu$m, de préférence allant de 20 nm à 50 $\mu$m.

9. Composition selon l'une quelconque des revendications précédentes, **caractérisée par le fait que** la composition comprend les premières particules solides et les deuxièmes particules solides.

10. Composition selon l'une des revendications précédentes, **caractérisée par le fait que** les deuxièmes particules solides sont choisies parmi :

   - des deuxièmes particules solides primaires comprenant un matériau amorphe ayant une température de transition vitreuse supérieure ou égale à 60 °C,
   - des deuxièmes particules solides secondaires, différentes desdites deuxièmes particules primaires,
   - et leurs mélanges.

11. Composition selon l'une quelconque des revendications précédentes, **caractérisée par le fait que** la fraction volumique des premières particules solides et, le cas échéant, des deuxièmes particules solides va de 60 % à 99 % du volume total de la fraction non volatile de la composition.

12. Composition selon la revendication précédente, **caractérisée par le fait que** la fraction volumique des premières particules solides et, le cas échéant, des deuxièmes particules solides est supérieure ou égale 70 %, de préférence va de 70 % à 95 %, du volume total de la fraction non volatile de la composition.

13. Composition selon l'une quelconque des revendications précédentes, **caractérisée par le fait que** la fraction volumique des premières particules solides représente de 30 % à 100 %, de la fraction volumique totale des premières et deuxièmes particules solides.

14. Composition selon l'une quelconque des revendications précédente, **caractérisée par le fait que** les deuxièmes particules solides comprennent des deuxièmes particules solides primaires comprenant un matériau amorphe solide à 25 °C présentant une transition vitreuse supérieure ou égale à 60 °C.

15. Composition selon la revendication précédente, **caractérisée par le fait que** les deuxièmes particules solides primaires comprennent un matériau amorphe ayant une température de transition vitreuse supérieure ou égale à 80 °C, et de préférence supérieure ou égale à 100 °C.

16. Composition selon l'une des revendications 14 ou 15, **caractérisée par le fait que** le matériau amorphe est un polymère.

17. Composition selon l'une quelconque des revendications 14 à 16, **caractérisée par le fait que** le matériau amorphe est un polymère choisi parmi les polymères radicalaires et les polycondensats.

18. Composition selon l'une quelconque des revendications 14 à 17, **caractérisée par le fait que** le matériau amorphe est un polymère choisi parmi les polymères d'éthylène, les polymères de propylène, les polymères acryliques, les polymères d'acrylamide, les polymères de (méth)acrylonitrile, les polycarbonates, les polyuréthanes, les polyesters, les polyamides, les polysulfones, les polysulfonamides, les carbohydrates.

19. Composition selon l'une quelconque des revendications 14 à 19, **caractérisée par le fait que** les deuxièmes particules solides primaires ont une taille moyenne allant de 10 nm à 50 $\mu$m, et de préférence allant de 20 nm à 1 $\mu$m.

20. Composition selon l'une quelconque des revendications 14 à 20, **caractérisée par le fait que** la fraction volumique des premières particules solides et des deuxièmes particules solides primaires, est supérieure ou égale à 10,05 % du volume total des premières et deuxièmes particules solides, avantageusement supérieure ou égale à 20,05 %, et de préférence supérieure ou égale à 30,05 %, préférentiellement supérieure ou égale à 40,05 %, et mieux supérieure ou égale à 50 %.

21. Composition selon l'une quelconque des revendications précédentes, **caractérisée par le fait que** les deuxièmes particules solides comprennent des deuxièmes particules solides secondaires.

22. Composition selon l'une quelconque des revendications précédentes, **caractérisée par le fait que** le solvant volatil est choisi parmi l'eau, les solvants organiques volatiles, les huiles volatiles, et leurs mélanges.

**23.** Composition selon l'une quelconque des revendications précédentes, **caractérisée par le fait que** le polymère apte à adhérer sur les matières kératiniques est choisi dans le groupe formé par les polymères vinyliques, les polyuréthanes, les polyesters, les polyamides, les polyurées, les polymères cellulosiques.

**24.** Composition selon l'une quelconque des revendications précédentes, **caractérisée par le fait que** le polymère apte à adhérer sur les matières kératiniques est un polymère filmogène à une température inférieure ou égale à 40 °C.

**25.** Composition selon l'une quelconque des revendications précédentes, **caractérisée par le fait que** le polymère apte à adhérer sur les matières kératiniques est un polymère apte à former un dépôt produisant à une concentration de 7 % dans l'eau, une rétraction du stratum corneum isolé de plus de 1 % à 30°C sous une humidité relative de 40 %.

**26.** Composition selon la revendication précédente, **caractérisée par le fait que** la rétraction du stratum cornéum est de plus de 1,2 %, et mieux de plus de 1,5 %.

**27.** Composition selon l'une quelconque des revendications précédentes, **caractérisée par le fait que** polymère apte à adhérer sur les matières kératiniques est pré sent en une teneur en matière sèche allant de 0,1 % à 50 % en poids, par rapport au poids total de la composition, de préférence de 0,5 % à 40 % en poids, et mieux de 1 % à 30 % en poids.

**28.** Composition selon l'une quelconque des revendications précédentes, **caractérisée par le fait qu'**elle comprend une phase aqueuse.

**29.** Composition selon l'une quelconque des revendications précédentes, **caractérisée par le fait qu'**elle comprend une phase aqueuse formée d'eau ou d'un mélange d'eau et de solvant organique miscible à l'eau.

**30.** Composition selon la revendication précédente, **caractérisée par le fait que** le solvant organique miscible à l'eau est choisi parmi les monoalcools inférieurs ayant de 1 à 5 atomes de carbone, les glycols ayant de 2 à 8 atomes de carbone, les cétones en $C_3$-$C_4$, les aldéhydes en $C_2$-$C_4$.

**31.** Composition selon l'une des revendications 28 à 30, **caractérisée par le fait que** la phase aqueuse est présente en une teneur allant de 0,1 % à 98 % en poids, par rapport au poids total de la composition, de préférence allant de 1 % à 80 % en poids, et mieux allant de 5 % à 65 % en poids.

**32.** Composition selon l'une quelconque des revendications 28 à 31, **caractérisée par le fait que** le polymère apte à adhérer sur les matières kératiniques est solubilisé dans la phase aqueuse.

**33.** Composition selon l'une quelconque des revendications 28 à 32, **caractérisée par le fait que** le polymère apte à adhérer sur les matières kératiniques est sous forme de particules solides en dispersion aqueuse.

**34.** Composition selon l'une quelconque des revendications précédentes, **caractérisée par le fait qu'**elle comprend une huile volatile.

**35.** Composition selon la revendication précédente, **caractérisée par le fait que** l'huile volatile est choisie parmi les huiles hydrocarbonées, les huiles siliconées, les huiles fluorées, ou leurs mélanges.

**36.** Composition selon l'une des revendications 34 ou 35, **caractérisée par le fait que** l'huile volatile est présente en une teneur allant de 0,1 % à 98 % en poids, par rapport au poids total de la composition, de préférence allant de 1 % à 65 % en poids, et mieux allant de 5 % à 65 % en poids.

**37.** Composition selon l'une quelconque des revendications précédentes, **caractérisée par le fait qu'**elle comprend une huile non volatile.

**38.** Composition selon la revendication précédente, **caractérisée par le fait que** l'huile non volatile est présente en une teneur allant de 0,1 % à 50 % en poids, de préférence de 0,1 % à 30 % en poids, par rapport au poids total de la composition, et mieux de 0,1 % à 20 % en poids.

**39.** Composition selon l'une quelconque des revendications précédentes, **caractérisée par le fait que** le polymère apte à adhérer sur les matières kératiniques est solubilisé ou dispersé sous forme de particules stabilisées en surface dans une phase grasse liquide.

**40.** Composition selon l'une quelconque des revendications précédentes, **caractérisée par le fait qu'** elle comprend une cire.

**41.** Composition selon la revendication précédente, **caractérisée par le fait que** la cire a un point de fusion allant de 30 °C à 99 °C, de préférence allant de 45°C à 99 °C.

**42.** Composition selon l'une des revendications 40 à 41, **caractérisée par le fait que** la cire est sous forme de particules ayant une taille moyenne allant de 50 nm à 50 $\mu$m, et de préférence de 50 nm à 10 $\mu$m.

**43.** Composition selon l'une des revendications 40 à 42, **caractérisée par le fait que** la cire est présente en une teneur allant de 0,1 % à 50 % en poids, de préférence allant de 0,5 % à 30 % en poids, et mieux allant de 1 % à 20 % en poids, par rapport au poids total de la composition.

**44.** Composition selon l'une quelconque des revendications précédentes, **caractérisée par le fait qu'**elle comprend un tensioactif.

**45.** Composition selon l'une quelconque des revendications précédentes, **caractérisée par le fait qu'** elle comprend un additif choisi parmi les matières colorantes, les antioxydants, les charges, les conservateurs, les parfums, les neutralisants, les épaississants, les actifs cosmétiques, les filtres solaires, les agents de coalescence, les plastifiants.

**46.** Composition selon l'une quelconque des revendications précédentes, **caractérisée par le fait qu'**elle est un mascara.

**47.** Procédé de maquillage ou de soin non thérapeutique des fibres kératiniques, notamment des cils, comprenant l'application sur les fibres kératiniques d'une composition selon l'une quelconque des revendications 1 à 46.

**48.** Utilisation d'une composition de revêtement des fibres kératiniques selon l'une quelconque des revendications 1 à 46, pour recourber les fibres kératiniques, notamment les cils.

**49.** Utilisation de premières particules solides comprenant un premier matériau cristallin ou semi-cristallin solide à 25 °C présentant une transition de phase de premier ordre, de fusion ou de combustion, supérieure à 100 °C, et, éventuellement, de deuxièmes particules solides comprenant un deuxième matériau différent du premier matériau, les deuxièmes particules solides n'étant pas aptes à coalescer à une température inférieure ou égale à 40 °C, dans une composition de revêtement des fibres kératiniques, notamment un mascara, comprenant, dans un milieu cosmétiquement acceptable comprenant au moins un solvant volatil, une fraction non volatile comprenant un polymère apte à adhérer sur les matières kératiniques et comprenant lesdites premières et deuxièmes particules solides, les premières particules solides et, le cas échéant, les deuxièmes particules solides étant présentes dans la composition en une teneur telle que la fraction volumique des premières particules solides et, le cas échéant, des deuxièmes particules solides est supérieure ou égale 60 % du volume total de la fraction non volatile de la composition, et, le cas échéant, la fraction volumique des premières particules solides étant supérieure ou égale à 30 % de la fraction volumique totale des premières et deuxièmes particules solides, pour recourber les fibres kératiniques, notamment les cils.

**Claims**

**1.** Composition for coating keratinous fibres comprising, in a cosmetically acceptable medium comprising at least one volatile solvent having a non-zero vapour pressure, at ambient temperature and atmospheric pressure, a nonvolatile fraction comprising:

- a polymer capable of adhering to the keratinous materials,
- first solid particles comprising a first crystalline or semicrystalline material which is solid at 25°C exhibiting a first order phase transition, a melting transition or a combustion transition temperature, greater than 100°C,
- and optionally second solid particles comprising a second material different from the first material, the second solid particles not being capable of coalescing at a temperature of less than or equal to 40°C,

the first solid particles and, where appropriate, the second solid particles being present in the composition in an amount such that the volume fraction of the first solid particles and, where appropriate, of the second solid particles is greater than or equal to 60% of the total volume of the nonvolatile fraction of the composition, and, where appropriate, the volume fraction of the first solid particles being greater than or equal to 30% of the total volume fraction of the first and second solid particles.

2. Composition according to Claim 1, **characterized in that** the first crystalline or semicrystalline material has a first order phase transition greater than 120°C, preferably greater than 150°C.

3. Composition according to either of Claims 1 and 2,
**characterized in that** the first material has a Vicker hardness greater than or equal to 10, preferably ranging from 10 to 7500.

4. Composition according to any one of Claims 1 to 3,
**characterized in that** the first material has a Vicker hardness greater than or equal to 200, preferably ranging from 200 to 7500.

5. Composition according to any one of Claims 1 to 4,
**characterized in that** the first material has a Vicker hardness greater than or equal to 400, preferably ranging from 400 to 7500.

6. Composition according to any one of the preceding claims, **characterized in that** the first material is chosen from the group consisting of silica, glass, diamond, copper, boron nitride, ceramics, metal oxides, micas, polyamides, and mixtures thereof.

7. Composition according to Claim 6, **characterized in that** the first material is chosen from iron oxides.

8. Composition according to any one of the preceding claims, **characterized in that** the first solid particles have a mean size ranging from 5 nm to 50 $\mu$m, preferably ranging from 20 nm to 50 $\mu$m.

9. Composition according to any one of the preceding claims, **characterized in that** the composition comprises the first solid particles and the second solid particles.

10. Composition according to one of the preceding claims, **characterized in that** the second solid particles are chosen from:

- second primary solid particles, comprising an amorphous material having a glass transition temperature of greater than or equal to 60°C,
- second secondary solid particles which are different from the said second primary particles,
- and mixtures thereof.

11. Composition according to any one of the preceding claims, **characterized in that** the volume fraction of the first solid particles, and, where appropriate, of the second solid particles ranges from 60% to 99% of the total volume of the nonvolatile fraction of the composition.

12. Composition according to the preceding claim, **characterized in that** the volume fraction of the first solid particles, and, where appropriate, of the second solid particles is greater than or equal to 70%, preferably ranges from 70% to 95%, of the total volume of the nonvolatile fraction of the composition.

13. Composition according to any one of the preceding claims, **characterized in that** the volume fraction of the first solid particles represents from 30% to 100% of the total volume fraction of the first and second solid particles.

14. Composition according to any one of the preceding claims, **characterized in that** the second solid particles comprise second primary solid particles comprising an amorphous material which is solid at 25°C exhibiting a glass transition greater than or equal to 60°C.

15. Composition according to the preceding claim,
**characterized in that** the second primary solid particles comprise an amorphous material having a glass transition temperature greater than or equal to 80°C, and preferably greater than or equal to 100°C.

16. Composition according to one of Claims 14 or 15, **characterized in that** the amorphous material is a polymer.

17. Composition according to any one of Claims 14 to 16, **characterized in that** the amorphous material is a polymer chosen from free-radical polymers and polycondensates.

18. Composition according to any one of Claims 14 to 17, **characterized in that** the amorphous material is a polymer chosen from ethylene polymers, propylene polymers, acrylic polymers, acrylamide polymers, (meth)acxylonitrile polymers, polycarbonates,
polyurethanes, polyesters, polyamides, polysulphones, polysulphonamides and carbohydrates.

19. Composition according to any one of Claims 14 to 19, **characterized in that** the second primary solid particles have a mean size ranging from 10 nm to 50 $\mu$m, and preferably ranging from 20 nm to 1 $\mu$m.

20. Composition according to any one of Claims 14 to 20, **characterized in that** the volume fraction of the first solid particles and of the second primary solid particles is greater than or equal to 10.05% of the total volume of the first and second solid particles, advantageously greater than or equal to 20.05%, and preferably greater than or equal to 30.05%, preferably greater than or equal to 40.05%, and better still greater than or equal to 50%.

21. Composition according to any one of the preceding claims, **characterized in that** the second solid particles comprise second secondary solid particles.

22. Composition according to any one of the preceding claims, **characterized in that** the volatile solvent is chosen from water, volatile organic solvents, volatile oils and mixtures thereof.

23. Composition according to any one of the preceding claims, **characterized in that** the polymer capable of adhering to the keratinous materials is chosen from the group consisting of vinyl polymers, polyurethanes, polyesters, polyamides, polyureas and cellulose polymers.

24. Composition according to any one of the preceding claims, **characterized in that** the polymer capable of adhering to the keratinous materials is a film-forming polymer at a temperature of less than or equal to 40°C.

25. Composition according to any one of the preceding claims, **characterized in that** the polymer capable of adhering to the keratinous materials is a polymer capable of forming a deposit producing, at a concentration of 7% in water, a retraction of the isolated stratum corneum of more than 1% at 30°C at a relative humidity of 40%.

26. Composition according to the preceding claim, **characterized in that** the retraction of the stratum corneum is of more than 1.2%, and better still of more than 1.5%.

27. Composition according to any one of the preceding claims, **characterized in that** the polymer capable of adhering to the keratinous materials is present in a dry matter content ranging from 0.1% to 50% by weight, relative to the total weight of the composition, preferably from 0.5% to 40% by weight, and better still from 1% to 30% by weight.

28. Composition according to any one of the preceding claims, **characterized in that** it comprises an aqueous phase.

29. Composition according to any one of the preceding claims, **characterized in that** it comprises an aqueous phase which consists of water or a mixture of water and water-miscible organic solvent.

30. Composition according to the preceding claim,
**characterized in that** the water-miscible organic solvent is chosen from lower monoalcohols having from 1 to 5 carbon atoms, glycols having from 2 to 8 carbon atoms, $C_3$-$C_4$ ketones, $C_2$-$C_4$ aldehydes.

31. Composition according to one of Claims 28-30,
**characterized in that** the aqueous phase is present in an amount ranging from 0.1% to 98% by weight, relative to the total weight of the composition, preferably ranging from 1% to 80% by weight, and better still ranging from 5% to 65% by weight.

**32.** Composition according to any one of Claims 28 to 31, **characterized in that** the polymer capable of adhering to the keratinous materials is solubilized in the aqueous phase.

**33.** Composition according to any one of Claims 28 to 32, **characterized in that** the polymer capable of adhering to the keratinous materials is in the form of solid particles in aqueous dispersion.

**34.** Composition according to any one of the preceding claims, **characterized in that** it comprises a volatile oil.

**35.** Composition according to the preceding claim, **characterized in that** the volatile oil is chosen from hydrocarbon oils, silicone oils, fluorinated oils or mixtures thereof.

**36.** Composition according to either of Claims 34 and 35, **characterized in that** the volatile oil is present in an amount ranging from 0.1% to 98% by weight, relative to the total weight of the composition, preferably ranging from 1% to 65% by weight, and better still ranging from 5% to 65% by weight.

**37.** Composition according to any one of the preceding claims, **characterized in that** it comprises a nonvolatile oil.

**38.** Composition according to the preceding claim, **characterized in that** the nonvolatile oil is present in an amount ranging from 0.1% to 50% by weight, preferably from 0.1% to 30% by weight, relative to the total weight of the composition, and better still from 0.1 to 20% by weight.

**39.** Composition according to any one of the preceding claims, **characterized in that** the polymer capable of adhering to the keratinous materials is Solubilized or dispersed in the form of surface-stabilized particles in a liquid fatty phase.

**40.** Composition according to any one of the preceding claims, **characterized in that** it comprises a wax.

**41.** Composition according to the preceding claim, **characterized in that** the wax has a melting point ranging from 30°C to 99°C, preferably ranging from 45°C to 99°C.

**42.** Composition according to one of Claims 40 to 41, **characterized in that** the wax is in the form of particles having a mean size ranging from 50 nm to 50 $\mu$m, and preferably from 50 nm to 10 $\mu$m.

**43.** Composition according to one of Claims 40 to 42, **characterized in that** the wax is present in an amount ranging from 0.1% to 50% by weight, preferably ranging from 0-5% to 30% by weight, and better still ranging from 1% to 20% by weight, relative to the total weight of the composition.

**44.** Composition according to any one of the preceding claims, **characterized in that** it comprises a surfactant.

**45.** Composition according to any one of the preceding claims, **characterized in that** it comprises an additive chosen from colouring substances, antioxidants, fillers, preservatives, perfumes, neutralizing agents, thickeners, cosmetic active agents, sunscreens, coalescing agents and plasticizers.

**46.** Composition according to any one of the preceding claims, **characterized in that** it is a mascara.

**47.** Method for the application of make-up to or the nontherapeutic treatment of keratinous fibres, in particular the eyelashes, comprising the application to the keratinous fibres of a composition according to any one of Claims 1 to 46.

**48.** Use of a composition for coating keratinous fibres according to any one of Claims 1 to 46, for curling the keratinous fibres, in particular eyelashes.

**49.** Use of first solid particles comprising a first semicrystalline or crystalline material which is solid at 25°C exhibiting a first order phase transition, a melting transition or a combustion transition, greater than 100°C, and optionally of second solid particles comprising a second material different from the first material, the second solid particles not being capable of coalescing at a temperature of less than or equal to 40°C, in a composition for coating keratinous fibres, in particular a mascara, comprising, in a cosmetically acceptable medium comprising at least one volatile solvent, a nonvolatile fraction comprising a polymer capable of adhering to the keratinous materials and comprising the said first and second solid particles, the first solid particles and, where appropriate, the second solid particles being present in the composition in an amount such that the volume fraction of the first solid particles and, where appropriate, of the second solid particles is greater than or equal to 60% of the total volume of the nonvolatile fraction of the composition,

and, where appropriate, the volume fraction of the first solid particles being greater than or equal to 30% of the total volume fraction of the first and second solid particles,

for curling keratinous fibres, in particular eyelashes.

**Patentansprüche**

1. Zusammensetzung zum Überziehen von Keratinfasern, die in einem kosmetisch akzeptablen kosmetischen Medium, das mindestens ein flüchtiges Lösungsmittel mit einem bei Raumtemperatur und Atmosphärendruck von Null verschiedenen Dampfdruck enthält, einen nichtflüchtigen Anteil enthält, mit:

   - einem Polymer, das an den Keratinsubstanzen haften kann,
   - ersten festen Partikeln, die ein kristallines oder teilkristallines, bei 25 °C festes erstes Material umfassen, das eine Phasenübergangstemperatur erster Ordnung, Schmelztemperatur oder Verdampfungstemperatur, über 100 °C aufweist, und
   - gegebenenfalls zweiten festen Partikeln, die ein zweites Material umfassen, das von dem ersten Material verschieden ist, wobei die zweiten festen Partikel bei einer Temperatur von 40 °C oder darunter nicht zur Koaleszenz befähigt sind, wobei die ersten festen Partikel und gegebenenfalls die zweiten festen Partikel in der Zusammensetzung in einer solchen Menge enthalten sind, dass der Volumenanteil der ersten festen Partikel und gegebenenfalls der zweiten festen Partikel 60 % oder mehr als 60 % des Gesamtvolumens des nichtflüchtigen Anteils der Zusammensetzung beträgt,
   und wobei, gegebenenfalls, der Volumenanteil der ersten festen Partikel größer oder gleich 30 % des gesamten Volumenanteils der ersten und zweiten festen Partikel ist.

2. Zusammensetzung nach Anspruch 1, **dadurch gekennzeichnet, dass** das erste kristalline oder teilkristalline Material eine Phasenübergangstemperatur erster Ordnung über 120 °C und vorzugsweise über 150 °C besitzt.

3. Zusammensetzung nach einem der Ansprüche 1 oder 2, **dadurch gekennzeichnet, dass** das erste Material eine Vickers-Härte von mindestens 10 und vorzugsweise 10 bis 7 500 besitzt.

4. Zusammensetzung nach einem der Ansprüche 1 bis 3, **dadurch gekennzeichnet, dass** das erste Material eine Vickers-Härte von mindestens 200 und vorzugsweise 200 bis 7 500 besitzt.

5. Zusammensetzung nach einem der Ansprüche 1 bis 4, **dadurch gekennzeichnet, dass** das erste Material eine Vickers-Härte von mindestens 400 und vorzugsweise 400 bis 7 500 besitzt.

6. Zusammensetzung nach einem der vorhergehenden Ansprüche, **dadurch gekennzeichnet, dass** das erste Material unter Siliciumdioxid, Glas, Diamant, Kupfer, Bornitrid, Keramiken, Metalloxiden, Glimmern, Polyamiden und deren Gemischen ausgewählt ist.

7. Zusammensetzung nach Anspruch 6, **dadurch gekennzeichnet, dass** das erste Material unter den Eisenoxiden ausgewählt ist.

8. Zusammensetzung nach einem der vorhergehenden Ansprüche, **dadurch gekennzeichnet, dass** die ersten festen Partikel eine mittlere Größe von 5 nm bis 50 $\mu$m und vorzugsweise 20 nm bis 50 $\mu$m aufweisen.

9. Zusammensetzung nach einem der vorhergehenden Ansprüche, **dadurch gekennzeichnet, dass** die Zusammensetzung erste feste Partikel und zweite feste Partikel enthält.

10. Zusammensetzung nach einem der vorhergehenden Ansprüche, **dadurch gekennzeichnet, dass** die zweiten festen Partikel ausgewählt sind unter:

    - primären zweiten festen Partikeln, die ein amorphes Material mit einer Glasübergangstemperatur von größer oder gleich 60 °C umfassen,
    - sekundären zweiten festen Partikeln, die von den primären zweiten Partikeln verschieden sind,
    - und deren Gemischen.

**EP 1 277 457 B1**

11. Zusammensetzung nach dem vorhergehenden Anspruch, **dadurch gekennzeichnet, dass** der Volumenanteil der ersten festen Partikel und gegebenenfalls der zweiten festen Partikel im Bereich von 60 bis 99 % des Gesamtvolumens des nichtflüchtigen Anteils der Zusammensetzung liegt.

12. Zusammensetzung nach dem vorhergehenden Anspruch, **dadurch gekennzeichnet, dass** der Volumenanteil der ersten festen Partikel und gegebenenfalls der zweiten festen Partikel mindestens 70 % und vorzugsweise 70 bis 95 % des Gesamtvolumens des nichtflüchtigen Anteils der Zusammensetzung beträgt.

13. Zusammensetzung nach einem der vorhergehenden Ansprüche, **dadurch gekennzeichnet, dass** der Volumenanteil der ersten festen Partikel 30 bis 100 % des Gesamtvolumenanteils der ersten und zweiten festen Partikel ausmacht.

14. Zusammensetzung nach einem der vorhergehenden Ansprüche, **dadurch gekennzeichnet, dass** die zweiten festen Partikel primäre zweite feste Partikel umfassen, die ein amorphes, bei 25 °C festes Material mit einer Glasübergangstemperatur von größer oder gleich 60°C umfassen.

15. Zusammensetzung nach dem vorhergehenden Anspruch, **dadurch gekennzeichnet, dass** die primären zweiten festen Partikel ein amorphes Material mit einer Glasübergangstemperatur von mindestens 80 °C und vorzugsweise mindestens 100 °C umfassen.

16. Zusammensetzung nach einem der Ansprüche 14 oder 15, **dadurch gekennzeichnet, dass** das amorphe Material ein Polymer ist.

17. Zusammensetzung nach einem der Ansprüche 14 bis 16, **dadurch gekennzeichnet, dass** das amorphe Material ein Polymer ist, das unter den durch radikalische Polymerisation hergestellten Polymeren und den Polykondensaten ausgewählt ist.

18. Zusammensetzung nach einem der Ansprüche 14 bis 17, **dadurch gekennzeichnet, dass** das amorphe Material ein Polymer ist, das unter den Polymeren von Ethylen, Polymeren von Propylen, Acrylpolymeren, Acrylamidpolymeren, (Meth)acrylnitrilpolymeren, Polycarbonaten, Polyharnstoffen, Polyestern, Polyamiden, Polysulfonen, Polysulfonamiden und Kohlenhydraten ausgewählt ist.

19. Zusammensetzung nach einem der Ansprüche 14 bis 19, **dadurch gekennzeichnet, dass** die primären zweiten festen Partikel eine mittlere Größe von 10 nm bis 50 $\mu$m und vorzugsweise 20 nm bis 1 $\mu$m besitzen.

20. Zusammensetzung nach einem der Ansprüche 14 bis 20, **dadurch gekennzeichnet, dass** der Volumenanteil der ersten festen Partikel und der primären zweiten festen Partikel größer oder gleich 10,05 % des Gesamtvolumens der ersten und zweiten festen Partikel ist, vorzugsweise größer oder gleich 20,05 %, bevorzugt größer oder gleich 30,05 %, vorzugsweise größer oder gleich 40,05 % und noch besser größer oder gleich 50 %.

21. Zusammensetzung nach einem der vorhergehenden Ansprüche, **dadurch gekennzeichnet, dass** die zweiten festen Partikel sekundäre zweite feste Partikel umfassen.

22. Zusammensetzung nach einem der vorhergehenden Ansprüche, **dadurch gekennzeichnet, dass** das flüchtige Lösungsmittel unter Wasser, flüchtigen organischen Lösungsmitteln, flüchtigen Ölen und deren Gemischen ausgewählt ist.

23. Zusammensetzung nach einem der vorhergehenden Ansprüche, **dadurch gekennzeichnet, dass** das Polymer, das befähigt ist, an den Keratinsubstanzen zu haften, unter den Vinylpolymeren, Polyurethanen, Polyestern, Polyamiden, Polyharnstoffen und Cellulosepolymeren ausgewählt ist.

24. Zusammensetzung nach einem der vorhergehenden Ansprüche, **dadurch gekennzeichnet, dass** das Polymer, das an den Keratinsubstanzen haften kann, ein Polymer ist, das bei einer Temperatur von 40 °C oder darunter filmbildend ist.

25. Zusammensetzung nach einem der vorhergehenden Ansprüche, **dadurch gekennzeichnet, dass** das Polymer, das befähigt ist, an den Keratinsubstanzen zu haften, ein Polymer ist, das eine Abscheidung bilden kann, die in einer Konzentration von 7 % in Wasser bei 30 °C und einer relativen Feuchte von 40 % eine Retraktion von isoliertem Stratum corneum von mehr als 1 % hervorruft.

26. Zusammensetzung nach dem vorhergehenden Anspruch, **dadurch gekennzeichnet, dass** die Retraktion des Stratum corneums mehr als 1,2 % und besser mehr als 1,5 % beträgt.

27. Zusammensetzung nach einem der vorhergehenden Ansprüche, **dadurch gekennzeichnet, dass** das Polymer, das befähigt ist, an den Keratinsubstanzen zu haften, in einem Trockensubstanzgehalt von 0,1 bis 50 Gew.-%, bezogen auf das Gesamtgewicht der Zusammensetzung, vorzugsweise 0,5 bis 40 Gew.-% und besser 1 bis 30 Gew.-% enthalten ist.

28. Zusammensetzung nach einem der vorhergehenden Ansprüche, **dadurch gekennzeichnet, dass** sie eine wässrige Phase enthält.

29. Zusammensetzung nach einem der vorhergehenden Ansprüche, **dadurch gekennzeichnet, dass** sie eine wässrige Phase enthält, die aus Wasser oder einem Gemisch von Wasser und einem organischen, mit Wasser mischbaren Lösungsmittel gebildet ist.

30. Zusammensetzung nach dem vorhergehenden Anspruch, **dadurch gekennzeichnet, dass** das organische, mit Wasser mischbare Lösungsmittel unter den niederen Monoalkoholen mit 1 bis 5 Kohlenstoffatomen, Glycolen mit 2 bis 8 Kohlenstoffatomen, $C_{3-4}$-Ketonen und $C_{2-4}$-Aldehyden ausgewählt ist.

31. Zusammensetzung nach einem der Ansprüche 28 bis 30, **dadurch gekennzeichnet, dass** die wässrige Phase in einer Menge von 0,1 bis 98 Gew.-%, bezogen auf das Gesamtgewicht der Zusammensetzung, vorzugsweise 1 bis 80 Gew.-% und besser 5 bis 65 Gew.-% enthalten ist.

32. Zusammensetzung nach einem der Ansprüche 28 bis 31, **dadurch gekennzeichnet, dass** das Polymer, das an den Keratinsubstanzen haften kann, in der wässrigen Phase gelöst ist.

33. Zusammensetzung nach einem der Ansprüche 28 bis 32, **dadurch gekennzeichnet, dass** das Polymer, das befähigt ist, an den Keratinsubstanzen zu haften, in Form von festen Partikeln in wässriger Dispersion vorliegt.

34. Zusammensetzung nach einem der vorhergehenden Ansprüche, **dadurch gekennzeichnet, dass** sie ein flüchtiges Öl enthält.

35. Zusammensetzung nach dem vorhergehenden Anspruch, **dadurch gekennzeichnet, dass** das flüchtige Öl unter den Kohlenwasserstofföreln, Siliconölen, fluorierten Ölen oder deren Gemischen ausgewählt ist.

36. Zusammensetzung nach einem der Ansprüche 34 oder 35, **dadurch gekennzeichnet, dass** das flüchtige Öl in einer Menge von 0,1 bis 98 Gew.-%, bezogen auf das Gesamtgewicht der Zusammensetzung, vorzugsweise 1 bis 65 Gew.-% und besser 5 bis 65 Gew.-% enthalten ist.

37. Zusammensetzung nach einem der vorhergehenden Ansprüche, **dadurch gekennzeichnet, dass** sie ein nichtflüchtiges Öl enthält.

38. Zusammensetzung nach dem vorhergehenden Anspruch, **dadurch gekennzeichnet, dass** das nichtflüchtige Öl in einer Menge von 0,1 bis 50 Gew.-%, vorzugsweise 0,1 bis 30 Gew.-%, bezogen auf das Gesamtgewicht der Zusammensetzung, und besser 0, 1 bis 20 Gew.-% enthalten ist.

39. Zusammensetzung nach einem der vorhergehenden Ansprüche, **dadurch gekennzeichnet, dass** das Polymer, das befähigt ist, an den Keratinsubstanzen zu haften, in Form von an der Oberfläche stabilisierten Partikeln in einer flüssigen Fettphase gelöst oder dispergiert ist.

40. Zusammensetzung nach einem der vorhergehenden Ansprüche, **dadurch gekennzeichnet, dass** sie ein Wachs enthält.

41. Zusammensetzung nach dem vorhergehenden Anspruch, **dadurch gekennzeichnet, dass** das Wachs einen Schmelzpunkt von 30 bis 99 °C und vorzugsweise von 45 bis 99 °C aufweist.

**42.** Zusammensetzung nach einem der Ansprüche 40 bis 41, **dadurch gekennzeichnet, dass** das Wachs in Form von Partikeln mit einer mittleren Größe von 50 nm bis 50 $\mu$m und vorzugsweise 50 nm bis 10 $\mu$m vorliegt.

**43.** Zusammensetzung nach einem der Ansprüche 40 bis 42, **dadurch gekennzeichnet, dass** das Wachs in einer Menge von 0, 1 bis 50 Gew.-%, vorzugsweise 0,5 bis 30 Gew.-% und besser 1 bis 20 Gew.-%, bezogen auf das Gesamtgewicht der Zusammensetzung, enthalten ist.

**44.** Zusammensetzung nach einem der vorhergehenden Ansprüche, **dadurch gekennzeichnet, dass** sie einen grenzflächenaktiven Stoff enthält.

**45.** Zusammensetzung nach einem der vorhergehenden Ansprüche, **dadurch gekennzeichnet, dass** sie einen Zusatzstoff enthält, der unter den Farbmitteln, Antioxidantien, Füllstoffen, Konservierungsmitteln, Parfums, Neutralisationsmitteln, Verdickungsmitteln, kosmetischen Wirkstoffen, Sonnenschutzfiltern, Koaleszenzmitteln und Weichmachern ausgewählt ist.

**46.** Zusammensetzung nach einem der vorhergehenden Ansprüche, **dadurch gekennzeichnet, dass** es sich um eine Mascara handelt.

**47.** Verfahren zum Schminken oder für die nichttherapeutische Pflege von Keratinfasern, insbesondere Wimpern, das das Aufbringen einer Zusammensetzung nach einem der Ansprüche 1 bis 46 auf die Keratinfasern umfasst.

**48.** Verwendung einer Zusammensetzung zum Überziehen von Keratinfasern nach einem der Ansprüche 1 bis 46, um Keratinfasern und insbesondere Wimpern zu krümmen.

**49.** Verwendung von ersten festen Partikeln, die ein erstes kristallines oder teilkristallines, bei 25 °C festes Material mit einem Phasenübergang erster Ordnung, beim Schmelzen oder Verdampfen, über 100°C, und gegebenenfalls zweite feste Partikel umfassen, die ein zweites, von dem ersten Material verschiedenes Material enthalten, wobei die zweiten festen Partikel bei einer Temperatur von 40 °C oder darunter nicht zur Koaleszenz befähigt sind,
in einer Zusammensetzung zum Überziehen von Keratinfasern, insbesondere einer Mascara, die in einem kosmetisch akzeptablen Medium, das mindestens ein flüchtiges Lösungsmittel enthält, einen nichtflüchtigen Anteil aufweist, der ein Polymer, das an den Keratinfasern haften kann und die ersten und zweiten festen Partikel enthält, wobei die ersten festen Partikel und gegebenenfalls die zweiten festen Partikel in der Zusammensetzung in einer solchen Menge enthalten sind, dass der Volumenanteil der ersten festen Partikel und gegebenenfalls der zweiten festen Partikel größer oder gleich 60 % des Gesamtvolumens des nichtflüchtigen Anteils der Zusammensetzung ist, und, gegebenenfalls, der Volumenanteil der ersten festen Partikel größer oder gleich 30 % des Gesamtvolumenanteils der ersten und zweiten festen Partikel ist,
um die Keratinfasern und insbesondere die Wimpern zu krümmen.

## RÉFÉRENCES CITÉES DANS LA DESCRIPTION

**Documents brevets cités dans la description**

- EP 1082953 A
- FR 2794970
- WO 9823251 A
- EP 749747 A
- EP 749746 A
- EP 923928 A
- EP 930060 A
- FR 2232303 A
- EP 847752 A
- EP 557196 A
- EP 1048282 A

**Littérature non-brevet citée dans la description**

- Encyclopedia of Chemical Technology. KIRK-OTH-MER. WILEY, 1979, vol. 22, 333-432